# EUROPÄISCHE PATENTANMELDUNG

(11) **EP 1 980 273 A1**
(43) Veröffentlichungstag der Anmeldung: **15.10.2008**
(21) Anmeldenummer: 07006968.7
(22) Anmeldetag: 03.04.2007
(51) Int. Cl.: A61L 2/07

(54) **Entkeimungsvorrichtung und Verfahren zum Desinfizieren von Prothesenlinern**

(71) Anmelder: DBK David + Baader GmbH, 76870 Kandel (DE)
(72) Erfinder: Wiener, Manfred, 67160 Salmbach (FR); Schäffner, Wolfgang, 76773 Kuhardt (DE); Biro, Zeev, 42781 Haan (DE)
(74) Vertreter: Grünecker, Kinkeldey, Stockmair & Schwanhäusser Anwaltssozietät

(57) **Zusammenfassung**

Die vorliegende Erfindung bezieht sich auf eine Entkeimungsvorrichtung zum Desinfizieren von Prothesenlinern. Die vorliegende Erfindung bezieht sich außerdem auf ein zugehöriges Verfahren zum Desinfizieren von Prothesenlinern. Die Entkeimungsvorrichtung zum Desinfizieren von Prothesenlinern umfasst eine Dampferzeugungseinheit (102), die eine elektrische Heizvorrichtung (112) aufweist und betrieben werden kann, um mittels der elektrischen Heizvorrichtung Wasserdampf (108) zu erzeugen, und eine Dampfführungseinheit (104), die mit der Dampferzeugungseinheit (102) gekoppelt ist, um den erzeugten Dampf in einen Innenbereich des Prothesenliners (106) zu führen.

## Beschreibung

Die vorliegende Erfindung bezieht sich auf eine Entkeimungsvorrichtung zum Desinfizieren von Prothesenlinern. Die vorliegende Erfindung bezieht sich außerdem auf ein zugehöriges Verfahren zum Desinfizieren von Prothesenlinern.

Hervorgerufen durch Unfälle und Krankheiten kann die Amputation einer Gliedmaße, insbesondere eines Teils eines Beins, erforderlich sein und nach einer solchen schwerwiegenden Operation werden Prothesen verwendet, um den Betroffenen einen Teil ihrer Beweglichkeit zurückzugeben.

Eine derartige Prothese umfasst dabei zwei wesentliche Bestandteile: die eigentliche künstliche Gliedmaße und einen so genannten Prothesenliner, der meist auch nur als Liner bezeichnet wird. Dieser Liner ist eine Art Strumpf mit einer Innenschicht aus Silikon und häufig einer Außenschicht aus einem Stoffgewebe. Der Liner wird direkt auf den Stumpf aufgeschoben und soll dabei möglichst eng anliegen. Er dient als Kopplungselement zwischen der Prothese und dem Stumpf.

Die weichelastische Hülse eines derartigen Prothesenliners, wie er beispielsweise in der WO 2007/003361 A2 offenbart ist, hat neben polsternden und stoßdämpfenden Wirkungen vor allem die Aufgabe, den Stumpf luftdicht zu umschließen, so dass der Liner nicht vom Stumpf abgezogen werden kann, und daher eine Prothese über eine mechanische Verbindung mit dem Liner und damit mit dem Stumpf sicher verbunden werden kann. Dafür muss sich die weichelastische Hülse eines solchen Liners eng an die Haut des Trägers anschmiegen, nachdem sie durch Umstülpen auf dem Stumpf des Trägers unter Vermeidung jeglichen Lufteinschlusses angebracht worden ist. In der Regel ist es also erwünscht, dass das Linermaterial direkten Kontakt zu der Haut des Prothesenträgers hat.

Es versteht sich, dass zwischen der Haut und dem Liner zwangsweise ein feuchtwarmes Milieu ausgebildet wird, welches die Vermehrung von Bakterien und Pilzen begünstigt. Darüber hinaus verschmutzt der Liner, hervorgerufen durch Hautschweiß, die natürliche Hautverschuppung oder auch durch äußere Einflüsse wie Cremes oder eindringenden Staub. Bei einer derartigen Verschmutzung zusammen mit dem auftretenden feuchtwarmen Milieu im Innenbereich besteht für den Träger die Gefahr einer Verkeimung mit schwerwiegenden Folgen für die Haut und insbesondere für die vernarbte Amputationsstelle. Aus diesem Grund muss jeder Amputierte den Liner innen frei von Bakterien und Pilzen halten, die zu Irritationen oder Entzündungen der Haut führen könnten.

Üblicherweise erfolgt die Reinigung und Entkeimung der Liner durch Auswaschen mit keimtötenden Substanzen. Solche keimtötenden Substanzen sind Chemikalien, die ihrerseits selbst Reizungen der Haut oder andere unerwünschte Nebenwirkungen verursachen können. Ein weiterer Nachteil bei dieser Reinigung ist, dass häufig gerade der Endbereich des Liners, also der Bereich, in welchem die Amputationsnarbe liegt, schwer zugänglich sein kann, was eine nur unzureichende Desinfektion ermöglicht.

Deshalb wird in der WO 2007/003361 vorgeschlagen, als Innenmaterial für den Prothesenliner ein weichelastisches Kunststoffmaterial mit antibakterieller und/oder fungizider Wirkung zu verwenden.

Diese Lösung hat aber zum Einen den Nachteil, dass bestehende Herstellungsverfahren für derartige Liner abgeändert werden müssen und vorhandene Liner für diese Verbesserung nicht nachrüstbar sind, dass zum Anderen aber auch ein zusätzliches Allergierisiko aufgrund der eingesetzten Chemikalien besteht. Darüber hinaus ist eine bekannte Tatsache, dass die antibakterielle und/oder fungizide Wirkung derartiger chemischer Zusätze im Laufe der Zeit nachlässt.

Die Aufgabe, die der vorliegenden Erfindung zugrunde liegt, besteht daher darin, eine Vorrichtung und ein Verfahren anzugeben, wodurch eine möglichst effektive Entkeimung jeder Art von Prothesenliner ohne zusätzliche Allergierisiken auf kostengünstige Weise erzielt werden kann.

Diese Aufgabe wird durch den Gegenstand der unabhängigen Patentansprüche gelöst. Vorteilhafte Weiterbildungen der vorliegenden Erfindung sind Gegenstand der abhängigen Patentansprüche.

Dabei basiert die vorliegende Erfindung auf der Idee, einen Prothesenliner mittels Wasserdampf zu entkeimen. Hierfür wird eine bestimmte Wassermenge von einer Dampferzeugungseinheit, die eine elektrische Heizvorrichtung umfasst, verdampft und es wird der Dampf mit Hilfe einer Dampfführungseinheit, die mit der Dampferzeugungseinheit gekoppelt ist, ins Innere des Liners eingeleitet. Durch die Temperatur des Dampfes werden die gesundheitsgefährdenden Keime abgetötet. Dabei ist bekannt, dass eine Temperatur von ca. 80° C in diesem Bereich für die ausreichende Keimabtötung genügt. Die Verwendung von Wasserdampf bietet im Vergleich zur Verwendung von trockener Heißluft den Vorteil, dass gleichzeitig eine mechanische Innenreinigung, also im Prinzip eine Art Auswaschen von vorhandenen Belägen auftritt.

Die erfindungsgemäße Anordnung umfasst im Wesentlichen zwei Grundelemente, nämlich eine Dampferzeugungseinheit und eine Dampfführungseinheit, die auch als Dampfverteiler bezeichnet werden kann. Die Dampferzeugungseinheit entspricht dabei im Wesentlichen dem Aufbau bekannter Vaporisatoren, wie sie beispielsweise aus der EP 1 532 906 A1 bekannt sind. Erfindungsgemäß wird der in der Dampferzeugungseinheit generierte Wasserdampf mittels einer Dampfführungseinheit, die mit der Dampferzeugungseinheit gekoppelt ist, in den Innenbereich des Prothesenliners eingeleitet.

Diese Anordnung hat den Vorteil, dass bis in die kritischen Bereiche des Prothesenliners hinein eine sichere Desinfektion erreicht werden kann. Darüber hinaus lässt sich mit einer solchen Anordnung jeder beliebige Liner desinfizieren. Für einen Fachmann ist außerdem klar, dass die erfindungsgemäße Anordnung selbstverständlich auch zur Desinfektion anderer ähnlich geformter Hohlkörper, z. B. Babykostbehältern, genutzt werden kann.

Gemäß einer vorteilhaften Ausführungsform der vorliegenden Erfindung weist die Dampferzeugungseinheit eine in einem Bodenbereich einer Vertiefung angeordnete Heizfläche auf. Damit können äußerst geringe Wassermengen in äußerst energiesparender Weise in Dampf übergeführt werden.

Gemäß einer vorteilhaften Ausführungsform umfasst die Heizvorrichtung ein PTC-Heizmodul, wie dies beispielsweise aus der EP 1 532 906 A1 bekannt ist.

Bildet man die Dampfführungseinheit als starren Hohlkörper aus, der wenigstens teilweise von dem Prothesenliner aufnehmbar ist, so kann in vorteilhafter Weise der Dampf unmittelbar ins Innere geleitet werden und gleichzeitig kann die Dampfführungseinheit als Stütze und Halterung des weichelastischen Liners während des Desinfektionsvorgangs dienen.

Die Dampfführungseinheit kann gemäß einer vorteilhaften Weiterbildung eine Wandung mit mindestens einer Öffnung oder Düse aufweisen, durch die hindurch der Wasserdampf in den Innenbereich des Prothesenliners treten kann. Beispielsweise können eine oder mehrere Öffnungen in einem Endbereich der Dampfführungseinheit angeordnet sein, so dass der Dampf mit der höchsten Temperatur in den am weitesten innen gelegenen Bereich, der gleichzeitig auch der kritische Bereich ist, da er mit der Amputationsnarbe in Kontakt kommt, unmittelbar hineingeleitet wird. Alternativ oder zusätzlich können noch weitere Öffnungen vorgesehen sein, die auch die übrigen Innenbereiche des Liners mit Dampf versorgen.

Gemäß einer vorteilhaften Weiterbildung der vorliegenden Erfindung hat die Dampfführungseinheit eine Auffangwanne zum Auffangen kondensierten Wassers.

Damit kann sichergestellt werden, dass die an der Innenseite des Liners kondensierende Flüssigkeit definiert gesammelt wird. Diese Flüssigkeit kann anschließend entweder durch entsprechende Öffnungen wieder an die Heizvorrichtung zurückgekoppelt werden, oder aber auch lediglich gesammelt werden, um anschließend entsorgt zu werden. Die Rückkopplung an die Heizvorrichtung kann über entsprechende Filter erfolgen, damit ausgewaschene Schmutzpartikel und Keime nicht in den Verdampfungsbereich gelangen.

Entsprechend der Form des Stumpfes und der Position des Liners mit Bezug auf den übrigen Körper des Trägers muss die durch den Rand des Liners definierte Fläche nicht unbedingt rechtwinklig zu der Längsachse des Liners verlaufen. Es hat sich gezeigt, dass für Linerformen, bei denen die durch den Rand definierte Fläche einen Winkel mit der Längsachse des Liners einschließt, der deutlich von 90° abweicht, zu viel Dampf an der Seite des Liners mit der kürzeren Seitenwand austreten kann.

Gemäß einer vorteilhaften Weiterbildung der vorliegenden Erfindung kann daher die Dampfführungseinrichtung beweglich gelagert sein, um zwischen dem Rand des Liners und dem Auffangbereich im Wesentlichen umlaufend den gleichen möglichst geringen Abstand einzuhalten. Die Dampfführungseinheit kann dabei beispielsweise ein starrer Hohlkörper sein, der über ein Gelenk mit dem Auffangbereich gekoppelt ist. Dabei können alle bekannten Arten von Gelenken und unter anderem auch solche mit einer entsprechenden Arretierung eingesetzt werden.

Zum Beispiel kann die Schrägstellung über eine Rändelschraube oder ein Kugelgelenk mit Überwurfmutter winkelrichtig fixiert werden.

Um die erfindungsgemäße Vorrichtung an verschiedene Größen von Prothesenlinern anzupassen, kann die Dampfführungseinheit längenverstellbar ausgeführt sein. Damit ist eine besonders universell einsetzbare Ausführungsform realisiert. Beispielsweise kann die Dampfführungseinheit teleskopisch ausgeführt sein um die gewünschte Länge einzustellen.

Gemäß einer alternativen Ausführungsform der erfindungsgemäßen Entkeimungsvorrichtung kann eine Adapterhülse vorgesehen sein, die vor dem Desinfektionsvorgang auf den Prothesenliner aufgeschoben wird, um einen schräg stehenden Rand des Liners leichter ausgleichen zu können. Diese Adapterhülse ist vorzugsweise aus einem weichelastischen Material wie Silikon hergestellt.

Weiterhin kann die Problematik eines schräg geschnittenen Liners auch dadurch gelöst werden, dass ein vorzugsweise starrer Passring vorgesehen wird. Dieser Passring kann lose in die Auffangwanne eingelegt sein oder aber unmittelbar (und damit unverlierbar) an der Dampfführungseinheit angeformt sein. Insbesondere bei der Variante mit einem Passring kann ein ausreichender Zwischenraum vorgesehen werden, um ein restloses Entweichen der Feuchtigkeit während des Trocknungsvorgangs sicherzustellen.

Sowohl die Adapterhülse wie auch der Passring haben den Vorteil, dass die Standfestigkeit des Geräts in keiner Weise beeinträchtigt ist.

Falls vor der Trocknung des Liners auch eine Desinfektion des Außenbereichs gewünscht ist, kann gemäß einer vorteilhaften Weiterbildung die Desinfektionsvorrichtung einen Deckel oder eine Haube aufweisen, die den gesamten Liner in einer im Wesentlichen geschlossenen Dampfkammer aufnimmt.

Weiterhin kann die erfindungsgemäße Vorrichtung mit einem Gebläse oder auch Heißluftgebläse ausgestattet sein, um die Trocknung zu beschleunigen und auch an schwer zugänglichen Stellen im Inneren des Liners sicherzustellen.

Anhand der in den beiliegenden Zeichnungen dargestellten Ausgestaltungen wird die Erfindung im vorliegenden näher erläutert. Ähnliche oder korrespondierende Einzelheiten sind in den Figuren mit denselben Bezugszeichen versehen. Es zeigen:
- **Fig. 1**: eine Seitenansicht der erfindungsgemäßen Entkeimungsvorrichtung gemäß einer ersten Ausführungsform mit aufgeschobenem Prothesenliner;
- **Fig. 2**: die Anordnung aus Fig. 1, die um 90° gedreht ist mit abgenommenem Prothesenliner;
- **Fig. 3**: die Anordnung aus Fig. 1 in einer Schnittansicht;
- **Fig. 4**: die erfindungsgemäße Vorrichtung gemäß einer zweiten Ausführungsform;
- **Fig. 5**: die Anordnung aus Fig. 4 mit aufgeschobenem Prothesenliner in einer Schnittdarstellung;
- **Fig. 6**: die Ausführungsform der Fig. 4 in einer in 90° gedrehten Seitenansicht;
- **Fig. 7**: die erfindungsgemäße Vorrichtung gemäß einer weiteren Ausführungsform;
- **Fig. 8**: die erfindungsgemäße Vorrichtung gemäß einer weiteren Ausführungsform.

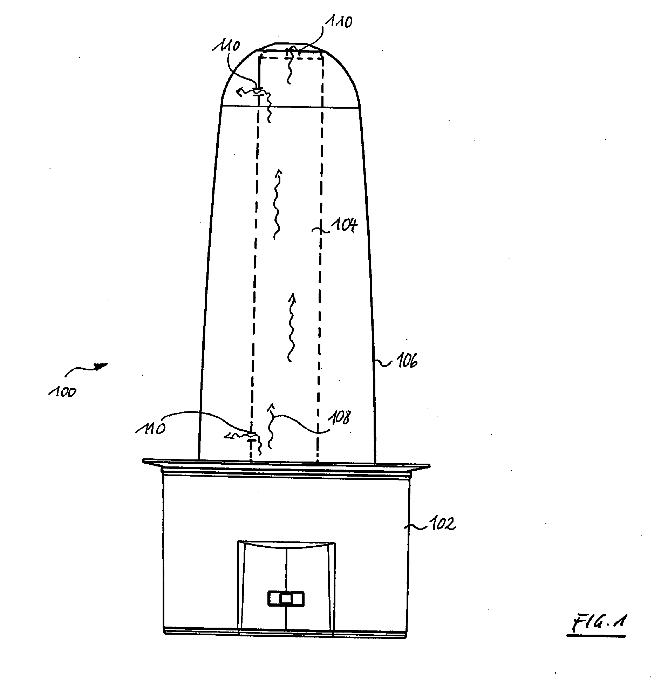

Fig. 1 zeigt in einer Seitenansicht die erfindungsgemäße Vorrichtung zum Desinfizieren von Prothesenlinern mit aufgesetztem Liner gemäß einer ersten Ausführungsform. Dabei umfasst die Desinfektionsvorrichtung 100 eine Dampferzeugungseinheit 102, die mittels eines elektrischen Heizelements eingefülltes Wasser in Wasserdampf überführt, sowie eine Dampfführungseinheit 104, um den Wasserdampf ins Innere des Prothesenliners 106 einzuleiten.

In ein Gehäuse wird von unten eine Heizung auf PTC-Basis eingebracht, welche das von oben eingefüllte Wasser verdampft. Neben der Heizung ist eine geeignete Schaltvorrichtung, z. B. ein Taster, vorgesehen. Zur Steuerung ist eine Elektronik vorgesehen, diese begrenzt die Laufzeit des Systems in an sich bekannter Weise. Neben der dargestellten Variante sind natürlich alle anderen Arten von elektrischen Heizsystemen möglich, z. B. Dickschichtheizungen, Drahtheizungen etc. Das erforderliche Netzkabel ist in den Figuren nicht dargestellt. Die Regelung kann außer mittels einer elektronischen Steuerung auch mit einem Thermostaten oder einem herkömmlichen Zeitschalter erfolgen, der auf verschiedene Zeiten einstellbar ist.

Erfindungsgemäß steigt, wie durch die Pfeile 108 dargestellt, der erzeugte Wasserdampf in der Dampfführungseinheit 104 nach oben und tritt über entsprechend angeordnete Dampfauslässe 110 aus der Dampferzeugungseinheit 104 aus. Die für den Dampfauslass vorgesehenen Öffnungen 110 sind dabei beispielhaft in ihrer Position. Sie können sowohl im Endbereich der Dampfführungseinheit 104, der mit dem innersten Bereich des Prothesenliners 106 in Kontakt kommt, angeordnet sein, alternativ aber auch in einem Bereich, welcher der Dampferzeugungseinheit 102 näher ist. Dadurch, dass die Dampfführungseinheit 104 in der gezeigten Ausführungsform als starrer Hohlkörper, hier als ein Rohr, ausgebildet ist, dient sie gleichzeitig als mechanische Haltevorrichtung für den Prothesenliner 106.

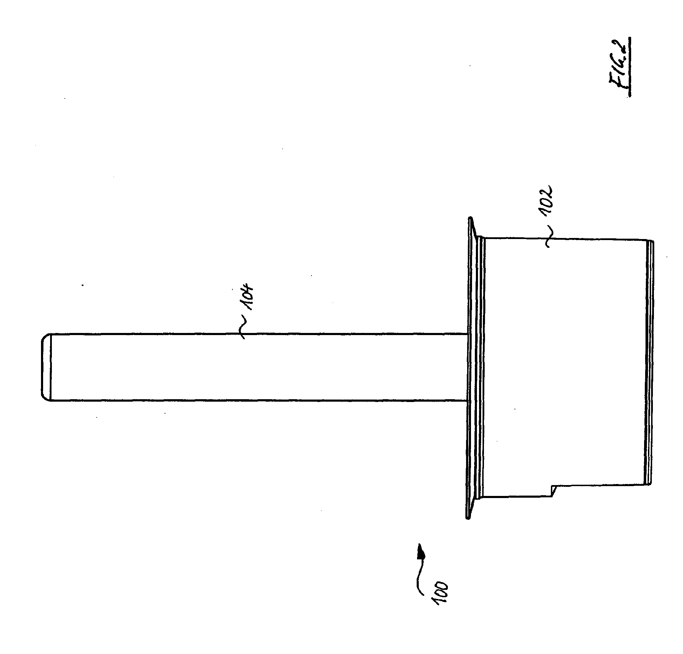

Eine um 90° gedrehte Ansicht der Desinfektionsvorrichtung gemäß Fig. 1 ist in Fig. 2 ohne den aufgeschobenen Liner 106 dargestellt.

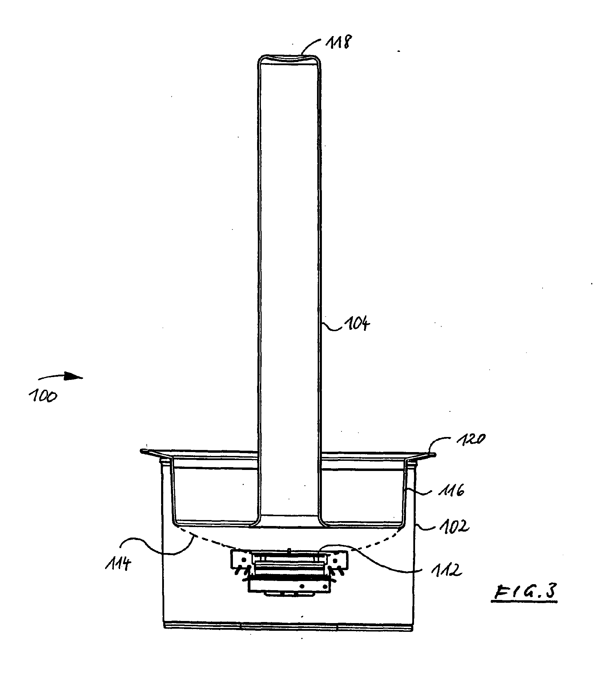

In Fig. 3 ist die Anordnung der Fig. 1 in einer Schnittdarstellung gezeigt. Wie aus dieser Darstellung erkennbar, weist die Dampferzeugungseinheit 102 eine Heizvorrichtung 112 auf, die in einem Bodenbereich einer Vertiefung 114 angeordnet ist. Die Dampfführungseinheit 104 ist in der hier gezeigten Ausführungsform als ein Rohr ausgebildet, das einstückig mit einer Auffangwanne 116 zum Auffangen des kondensierten Wassers ausgebildet ist. In der hier gezeigten Ausführungsform ist die aus der Auffangwanne 116 und dem Rohr 104 gebildete Einheit so ausgeführt, dass sie von der Dampferzeugungseinheit 102 abgenommen werden kann. Bei abgenommener Dampfführungseinheit kann in die Vertiefung 114 Wasser eingefüllt werden, das erfindungsgemäß von der Heizvorrichtung 112 verdampft wird. Das verdampfte Wasser steigt in der Dampfführungseinheit 104 nach oben und tritt an hier nicht dargestellten Dampfauslassöffnungen 110 nach außen. An dem aufgeschobenen Liner, der hier nicht dargestellt ist, kondensiert der Wasserdampf und tropft in die Auffangwanne 116 zurück.

In der Auffangwanne 116 können im Bodenbereich Öffnungen vorgesehen sein, die das kondensierte Wasser in die Vertiefung 116 zurückführen. Es ist allerdings zweckmäßig, hier eine Filterung vorzusehen, damit die Dampferzeugungseinheit 102 nicht verschmutzt wird. Alternativ kann das Wasser in der Auffangwanne 116 auch lediglich gesammelt werden, damit es später entsorgt wird. Der Vorteil der hier gezeigten Ausführungsform ist, dass die einstückige Anordnung aus Dampfführungseinheit 104 und Auffangwanne 116 auf einfache Weise entnommen und hygienisch gereinigt werden kann, wenn der Entkeimungsprozess abgeschlossen ist.

In der hier gezeigten Ausführungsform weist die Heizvorrichtung 112 ein PTC-Heizmodul analog zu der Anordnung aus der EP 1 532 906 A1 auf. Die Heizvorrichtung ist in dem Bodenbereich der Vertiefung 114 angeordnet, so dass nur wenig Wasser erforderlich ist um ausreichende Mengen an Wasserdampf zum Entkeimen eines Liners bereitzustellen.

Die erfindungsgemäße Dampfführungseinheit lässt sich somit als eine Art Teller mit einem zentral angeformten Dampfrohr betrachten. Wie bereits erwähnt, steigt der erzeugte Wasserdampf in dem Dampfrohr 104 nach oben und wird durch geeignete Düsen (die hier nicht dargestellt sind) nach außen geleitet. Diese Düsen (oder Dampfaustritte) können dabei ganz oben in einem Endbereich 118 oder aber auch seitlich in verschiedenen Höhen angeordnet sein. Der über dieses Dampfrohr gestülpte Prothesenliner wird somit gleichmäßig von Wasserdampf versorgt, wobei die Temperaturanforderung für eine ausreichende Keimabtötung 80° C beträgt. Bei der hier gezeigten Ausführungsform hängt im Betrieb der Prothesenliner über dem Dampfrohr, steht jedoch unten nicht auf dem Boden der Auffangwanne 116 auf, sondern hängt frei. Durch dieses freie Hängen ist eine Rückführung des kondensierenden Wasserdampfs zur Heizvorrichtung 112 hin möglich, indem beispielsweise das Wasser von dem Liner in die Auffangwanne 116 tropft und diese unten einige Öffnungen besitzt.

Eine weitere Folge des freien Hängens ist die Möglichkeit, dass der Liner nach dem Bedampfen austrocknen kann. Diese Trocknung ist erforderlich, da ein nasser Liner nicht auf den Stumpf aufgezogen werden sollte.

Gemäß einer weiteren Ausführungsform kann das Rohr 104 auch teleskopisch ausgebildet sein, um eine Höhenverstellung zum Adaptieren an die Linerlänge, beispielsweise mit einer Schraubmutter außen, zu ermöglichen.

Falls gewünscht ist, dass der Außenbereich des Liners ebenfalls mittels Dampf desinfiziert wird, kann eine Haube vorgesehen sein, die über die gesamte Anordnung gestellt wird und auf dem Auffangbeckenrand 120 aufsitzt. Wie bereits erwähnt, muss aber vor der eigentlichen Benutzung des Liners dieser dennoch noch durchtrocknen.

Weiterhin kann die erfindungsgemäße Entkeimungsvorrichtung mit einem Gebläse oder auch Heißluftgebläse ausgestattet sein, um die Trocknung zu beschleunigen und auch an schwer zugänglichen Stellen im Inneren des Liners sicherzustellen. Dieses Gebläse ist in den Figuren nicht dargestellt, kann aber in an sich bekannter Weise in dem Gehäuse der Dampferzeugungseinheit 102 untergebracht werden.

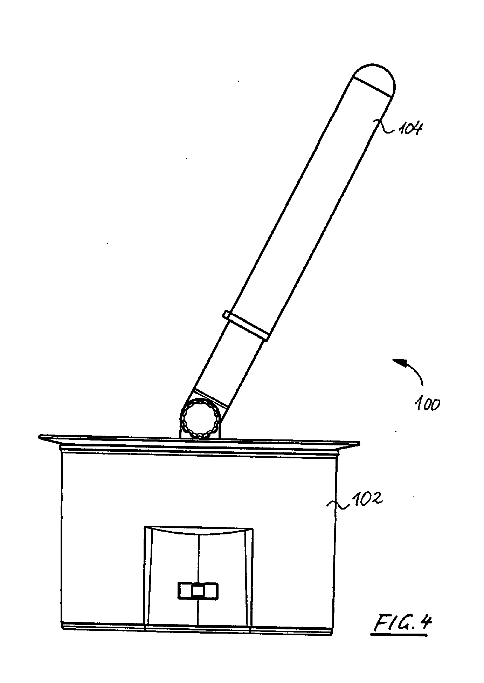

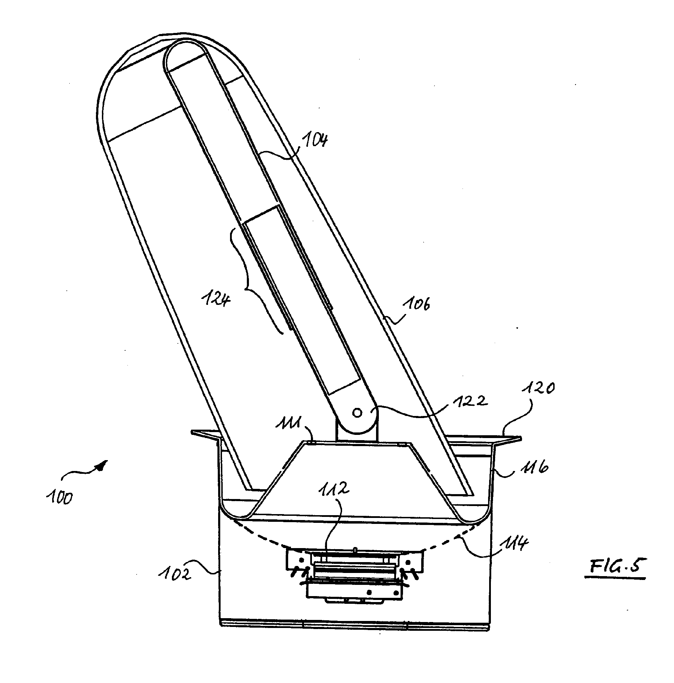

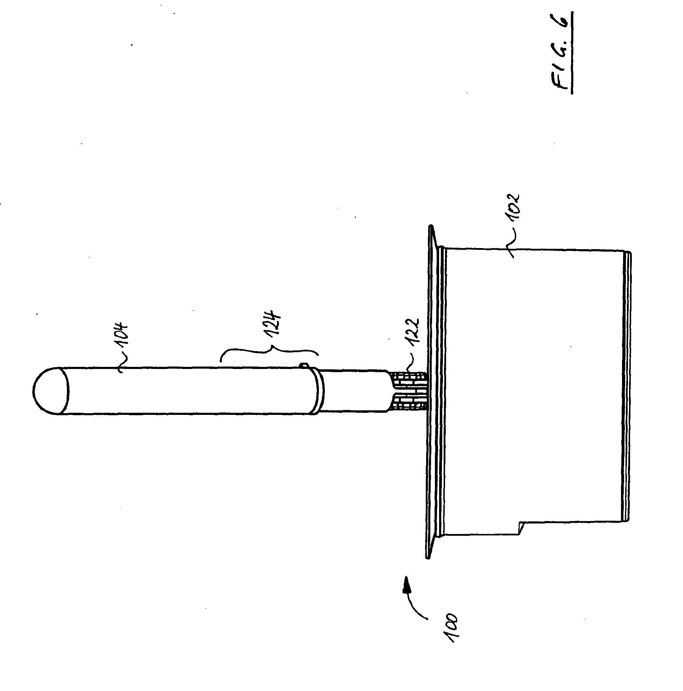

Eine zweite Ausführungsform der vorliegenden Erfindung soll im Folgenden unter Bezugnahme auf die Fig. 4 bis 6 und insbesondere auf die Schnittdarstellung der Fig. 5 erfolgen. Die erfindungsgemäße Desinfektionsvorrichtung 100 umfasst hier wiederum eine Dampferzeugungseinheit 102 und eine Dampfführungseinheit 104. Die Dampfführungseinheit ist an einer Auffangwanne 116 montiert. Im Unterschied zu den bisherigen Figuren ist jedoch die Dampfführungseinheit 104 nicht zentral und im Wesentlichen senkrecht zu einer durch die Auffangwanne 116 definierten Ebene angeordnet, sondern über ein Gelenk 122 beweglich.

Häufig ist nämlich das offene Ende des Prothesenliners nicht gerade, d. h. quer zur Längsachse abgeschnitten, sondern schräg geformt. Dadurch gibt sich das Problem, dass der Wasserdampf bei einem senkrecht ausgerichteten Rohr 104 schnell seitlich entweicht und sich deshalb im Inneren des Prothesenliners die geforderte Temperatur nicht mehr einstellt.

Die vorliegende zweite Ausführungsform löst dieses Problem, indem durch Kippen der Dampfführungseinheit 104, die hier beispielsweise als Rohr ausgebildet ist, mittels eines Gelenks 122 der Prothesenliner so angeordnet wird, dass sein offenes Ende umlaufend im Wesentlichen den gleichen Abstand zu der Auffangwanne 116 aufweist. Diese Schrägstellung bis zum horizontalen Verlauf des offenen Endes des Prothesenliners erfolgt gemäß der hier gezeigten Ausführungsform durch eine Verstellvorrichtung 122, welche das Zentralrohr, z. B. mittels einer Rändelschraube oder eines Kugelgelenks mit Überwurfmutter, winkelrichtig fixiert.

Gegebenenfalls muss noch eine Kippsicherung vorgesehen werden, welche z. B. an der Dampferzeugungseinheit 102, aber auch an der Auffangwanne 116 angeformt sein kann. Bei der hier gezeigten Ausführungsform kann der Dampfauslass aus an anderen Bereichen als dem Endbereich des Rohrs erfolgen, wie dies schematisch durch die Dampfauslässe 111 skizziert ist.

Weiterhin ist, wie durch den höhenverstellbaren Bereich 124 symbolisiert, die Dampfführungseinheit 104 teleskopisch ausgebildet. Mittels einer Klemmvorrichtung oder Überwurfmutter wird das übergeschobene Rohr an dem innen liegenden Rohr befestigt. Auf diese Weise kann die Länge der Dampfführungseinheit an die tatsächliche Länge des Prothesenliners angepasst werden.

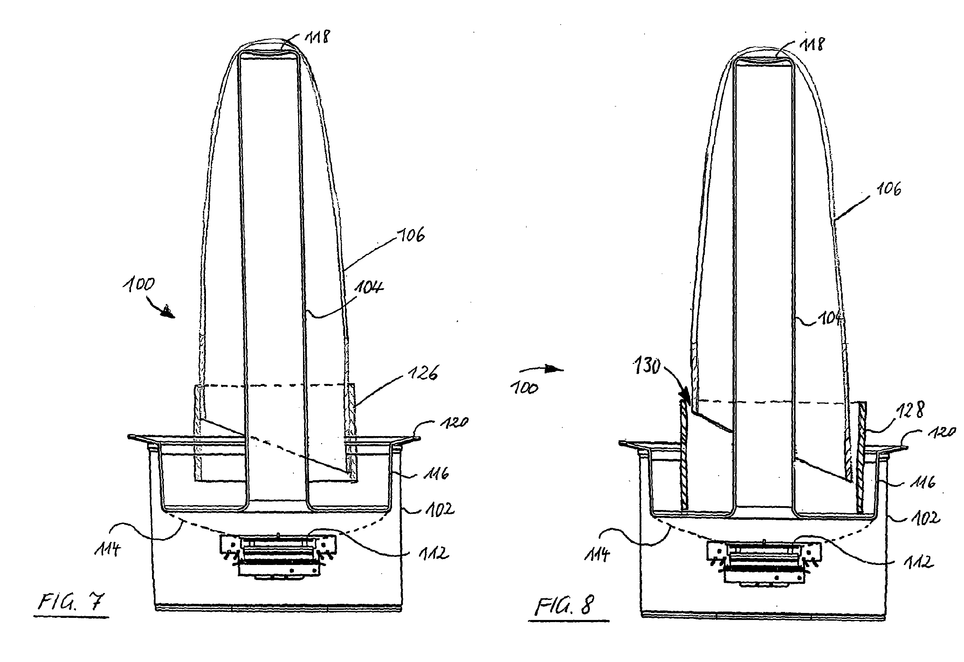

Eine weitere vorteilhafte Ausführungsform ist in Figur 7 dargestellt. Um die mit der zweiten Ausführungsform möglichen Probleme bezüglich der Standfestigkeit der Anordnung zu vermeiden, ist hier zum Kompensieren eines schräg geformten Prothesenlinerrandes eine Adapterhülse 126 vorgesehen, die über den Liner geschoben wird, bevor dieser auf die Dampfführungseinheit aufgesetzt wird. Diese Variante stellt eine besonders einfache und kostengünstige Lösung dar. Die Adapterhülse 126 kann vorzugsweise aus einem weichelastischen Material wie Silikon hergestellt sein.

Weiterhin kann die Problematik eines schräg geschnittenen Liners auch dadurch gelöst werden, dass ein vorzugsweise starrer Passring 128 vorgesehen wird, wie dies in Figur 8 gezeigt ist. Dieser Passring 128 kann lose in die Auffangwanne 116 eingelegt sein oder aber unmittelbar (und damit unverlierbar) an der Dampfführungseinheit 104 angeformt sein. Bei der in Figur 8 gezeigten Variante kann ein ausreichender Freiraum 130 vorgesehen werden, um ein restloses Entweichen der Feuchtigkeit während des Trocknungsvorgangs sicherzustellen.

Mit Hilfe der erfindungsgemäßen Vorrichtung lässt sich ohne zusätzliche Belastung durch Chemikalien eine sehr weitgehende Entkeimung von Prothesenlinern erreichen, da nur Wasserdampf als desinfizierendes Medium eingesetzt wird. Weiterhin kann die erfindungsgemäße Vorrichtung für alle Arten auch vorhandener Prothesenliner eingesetzt werden.

Es ist aber für einen Fachmann klar, dass die erfindungsgemäße Entkeimungsvorrichtung auch für andere Hohlkörper eingesetzt werden kann, für die eine Temperatur von 80° C im Innenraum zur Keimabtötung genügt. Beispielsweise können Gefäße wie Babyflaschen mit der erfindungsgemäßen Entkeimungsvorrichtung behandelt werden.

Die Vorgehensweise beim Desinfizieren von Prothesenlinern umfasst die folgenden Schritte:
Erzeugen von Wasserdampf mittels einer elektrischen Heizvorrichtung, und
Einleiten des erzeugten Dampfs in einen Innenbereich des Prothesenliners mittels einer Dampfführungseinheit , die mit der Dampferzeugungseinheit gekoppelt ist.

Weiterhin kann der Schritt des Einleitens von Luft und/oder Heißluft in einen Innenbereich des Prothesenliners zum Trocknen des Innenbereichs vorgesehen sein. Diese Luft bzw. Heißluft kann durch ein in dem Gerät integriertes (Heißluft)-Gebläse bereitgestellt werden.

## Patentansprüche

1. Entkeimungsvorrichtung zum Desinfizieren von Prothesenlinern mit einer Dampferzeugungseinheit (102), die eine elektrische Heizvorrichtung (112) umfasst und betrieben werden kann, um mittels der elektrischen Heizvorrichtung Wasserdampf (108) zu erzeugen, und
einer Dampfführungseinheit (104), die mit der Dampferzeugungseinheit (102) gekoppelt ist, um den erzeugten Dampf in einen Innenbereich des Prothesenliners (106) zu führen.

2. Vorrichtung nach Anspruch 1, wobei die Dampferzeugungseinheit (102) eine in einem Bodenbereich einer Vertiefung (114) angeordnete Heizfläche aufweist.

3. Vorrichtung nach Anspruch 1 oder 2, wobei die Heizvorrichtung ein PTC-Heizmodul aufweist.

4. Vorrichtung nach mindestens einem der vorangegangenen Ansprüche, wobei die Dampfführungseinheit (104) einen starren Hohlkörper, der wenigstens teilweise von dem Prothesenliner (106) aufnehmbar ist, umfasst.

5. Vorrichtung nach mindestens einem der vorangegangenen Ansprüche, wobei die Dampfführungseinheit (104) eine Wandung mit mindestens einer Öffnung (110), durch die hindurch Dampf treten kann, aufweist.

6. Vorrichtung nach Anspruch 5, wobei die mindestens eine Öffnung (110) in einem Endbereich (118) der Dampfführungseinheit (104) angeordnet ist.

7. Vorrichtung nach mindestens einem der vorangegangenen Ansprüche, wobei die Dampfführungseinheit eine Auffangwanne (116) zum Auffangen kondensierten Wassers aufweist.

8. Vorrichtung nach Anspruch 7, wobei die Auffangwanne (116) zum Zurückleiten des kondensierten Wassers an die Heizvorrichtung (112) mit der Dampferzeugungseinheit (102) gekoppelt ist.

9. Vorrichtung nach mindestens einem der vorangegangenen Ansprüche soweit abhängig von Anspruch 4, wobei der starre Hohlkörper bezüglich der Dampferzeugungseinheit (102) beweglich gelagert ist.

10. Vorrichtung nach Anspruch 9, wobei der starre Hohlkörper an einem Gelenk (122) gelagert ist.

11. Vorrichtung nach mindestens einem der vorangegangenen Ansprüche, wobei die Dampfführungseinheit (104) längeneinstellbar ausgeführt ist.

12. Vorrichtung nach Anspruch 11, wobei die Dampfführungseinheit (104) teleskopisch ausgeführt ist.

13. Vorrichtung nach mindestens einem der vorangegangenen Ansprüche, weiterhin umfassend eine Adapterhülse (126) zum Aufschieben auf den Prothesenliner (106).

14. Vorrichtung nach mindestens einem der vorangegangenen Ansprüche, weiterhin umfassend einen Passring (128), der so angeordnet ist, dass er im Betrieb einen offenen Randbereich des Prothesenliners (106) umgibt.

15. Vorrichtung nach mindestens einem der vorangegangenen Ansprüche, weiterhin umfassend eine Abdeckhaube, die so ausgeführt ist, dass der Prothesenliner (106) im Betrieb in einem im Wesentlichen geschlossenen Dampfraum eingeschlossen ist.

16. Vorrichtung nach mindestens einem der vorangegangenen Ansprüche, weiterhin umfassend einen Lüfter zum Einleiten von Luft in den Prothesenliner (106).

17. Vorrichtung nach Anspruch 16, wobei der Lüfter so ausgebildet ist, dass er Heißluft erzeugt.

18. Verfahren zum Desinfizieren von Prothesenlinern mit den folgenden Schritten:
Erzeugen von Wasserdampf mittels einer elektrischen Heizvorrichtung, und
Einleiten des erzeugten Dampfs in einen Innenbereich des Prothesenliners mittels einer Dampfführungseinheit, die mit der Dampferzeugungseinheit gekoppelt ist.

19. Verfahren nach Anspruch 18, weiterhin umfassend den Schritt:
Einleiten von Luft und/oder Heißluft in einen Innenbereich des Prothesenliners zum Trocknen des Innenbereichs.
